(19) Europäisches Patentamt
European Patent Office
Office européen des brevets

(11) Veröffentlichungsnummer: **0 008 076**
**A1**

(12) **EUROPÄISCHE PATENTANMELDUNG**

(21) Anmeldenummer: 79102759.2

(22) Anmeldetag: 01.08.79

(51) Int. Cl.³: C 07 C 177/00, C 07 C 47/267, C 07 D 319/06, A 61 K 31/557

(30) Priorität: 04.08.78 DE 2834249

(43) Veröffentlichungstag der Anmeldung: 20.02.80 Patentblatt 80/4

(84) Benannte Vertragsstaaten: AT BE CH DE FR GB IT NL SE

(71) Anmelder: HOECHST AKTIENGESELLSCHAFT, Zentrale Patentabteilung Postfach 80 03 20, D-6230 Frankfurt/Main 80 (DE)

(72) Erfinder: Bartmann, Wilhelm, Dr., Am Dachsbau 5, D-6232 Bad Soden am Taunus (DE)
Erfinder: Beck, Gerhard, Dr., Gustav-Freytag-Strasse 24, D-6000 Frankfurt am Main (DE)
Erfinder: Lerch, Ulrich, Dr., Schwalbenweg 19, D-6238 Hofheim am Taunus (DE)
Erfinder: Konz, Elmar, Dr., Buchenweg 22, D-6232 Bad Soden am Taunus (DE)

(54) **Neue Prostaglandinderivate in der Delta-2-11-Desoxy-PGF2- bzw. PGE2-Reihe, ihre Verwendung und Verfahren zu ihrer Herstellung und Verfahren zur Herstellung von Arzneimitteln.**

(57) Die vorliegende Erfindung betrifft neue Verbindungen der Formel I,

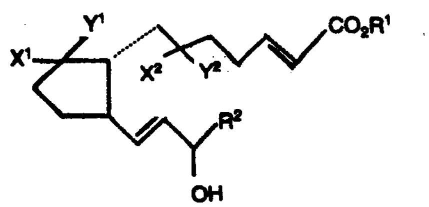

I

die strukturell mit den natürlichen Prostaglandinen verwandt sind, und in welcher bedeuten

$X^1$ und $Y^1$ sowie $X^2$ und $Y^2$ zusammen Sauerstoff oder jeweils Wasserstoff oder eine Hydroxylgruppe, wobei $X^1$ und $Y^1$ sowie $X^2$ und $Y^2$ verschieden sind,

$R^1$ a) Wasserstoff oder einen geradkettigen, verzweigten gesättigten oder ungesättigten aliphatischen oder cycloaliphatischen Kohlenwasserstoffrest mit bis zu zehn Kohlenstoffatomen oder

b) ein physiologisch verträgliches Metall-, oder $NH_4\pm$Ion oder ein Ammoniumion, das sich von einem primären, sekundären oder tertiären Amin ableitet,

$R^2$ einen geraden, verzweigten, gesättigten oder ungesättigten aliphatischen Kohlenwasserstoffrest mit zwei bis sieben Kohlenstoffatomen, in dem eine nicht endständige $CH_2$-Gruppe durch ein Sauerstoffatom ersetzt sein kann, oder ein Cycloalkylrest mit drei bis sieben Kohlenstoffatomen, z.B. die Verbindung 6,15-Dihydroxy-9-oxo-(E)-2, (E)-13-prostadiensäure, sowie ein Verfahren zu deren Herstellung. Die erfindungsgemäßen Verbindungen zeigen wertvolle pharmakologische, insbesondere blutplättchenaggregationshemmende und blutdrucksenkende Wirkungen und können daher als Arzneimittel verwendet werden.

EP 0 008 076 A1

BEZEICHNUNG GEÄNDERT
siehe Titelseite

HOECHST AKTIENGESELLSCHAFT HOE 78/F 161 Dr.LA/Rp

Neue Prostaglandinderivate in der $\Delta$ 2-11-Desoxy-$PGF_2$ bzw. $PGE_2$-Reihe, Verfahren zu ihrer Herstellung und ihre Verwendung als Arzneimittel

Prostaglandine sind eine Gruppe von Fettsäuren, die in zahlreichen Geweben und Organen von Mensch und Tier vorkommen. Das Grundgerüst der natürlichen Prostaglandine besteht aus 20 Kohlenstoffatomen, die in Form eines Fünfrings und zweier benachbarter linearer Seitenketten angeordnet sind.

Die pharmakologischen Effekte der Prostaglandine erstrecken sich u.a. auf die Gebiete der Reproduktion, des Bronchialmuskeltonus, des Blutdrucks und der Gastroenterologie. Die pharmakologischen Eigenschaften der natürlichen Prostaglandine sind Gegenstand zahlreicher Übersichtsartikel, z.B. N.H. Andersen und P.W. Ramwell in Arch. Internal Med. 133, 30 (1974); R.L. Jones in Pathobiology Ann. 1972, 359; J. Pike in Scient. American 225, 84 (1971) oder M.P.L. Caton in Progress in Med. Chem. vol. 8, ed: Butterworth, London, 1971.

Die Synthesen von nicht natürlich vorkommenden Analogen von Prostansäuren, in denen die Vielzahl der pharmakologischen Wirkungen der natürlichen Prostaglandine differenziert sind, gewinnt zunehmend an Bedeutung.

Die vorliegende Erfindung betrifft neue Verbindungen der Formel I

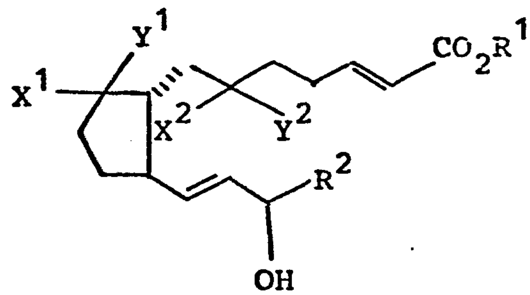

I

die strukturell mit den natürlichen Prostaglandinen verwandt sind undin welcher bedeuten
$X^1$ und $Y^1$ sowie $X^2$ und $Y^2$ zusammen Sauerstoff oder jeweils Wasserstoff oder eine Hydroxylgruppe, wobei $X^1$ und $Y^1$ sowie $X^2$ und $Y^2$ verschieden sind

$R^1$ a) Wasserstoff oder einen geradkettigen, verzweigten gesättigten oder ungesättigten aliphatischen oder cycloaliphatischen Kohlenwasserstoffrest mit bis zu zehn Kohlenstoffatomen oder

b) ein physiologisch verträgliches Metall-, oder $NH_4$ $\pm$ Ion oder ein Ammoniumion, das sich von einem primären, sekundären oder tertiären Amin ableitet,

$R^2$ einen geraden, verzweigten, gesättigten oder ungesättigten aliphatischen Kohlenwasserstoffrest mit zwei bis sieben Kohlenstoffatomen, in dem eine nicht endständige $CH_2$-Gruppe durch ein Sauerstoffatom ersetzt sein kann, oder ein Cycloalkylrest mit drei bis sieben Kohlenstoffatomen.

Bevorzugt beeinhaltet die vorliegende Erfindung Verbindungen der Formel I, worin bedeuten

$X^1$ und $Y^2$ sowie $X^2$ und $Y^2$ zusammen Sauerstoff oder jeweils Wasserstoff und eine Hydroxylgruppe, wobei $X^1$ und $Y^1$ sowie $X^2$ und $Y^2$ verschieden sind

$R^1$ a) Wasserstoff oder einen geradkettigen, verzweigten gesättigten aliphatischen oder cycloaliphatischen Kohlenwasserstoffrest mit bis zu sieben Kohlenstoffatomen oder

b) ein physiologisch verträgliches Metall- oder $NH_4^{\pm}$ Ion oder ein Ammoniumion, das sich von einem primären, sekundären oder tertiären Amin ableitet, und

$R^2$ einen geraden oder verzweigten, Alkylrest mit drei bis sieben Kohlenstoffatomen, in dem eine nicht endständige $CH_2$-Gruppe durch ein Sauerstoffatom ersetzt sein kann, oder ein Cycloalkylrest mit fünf bis sieben Kohlenstoffatomen.

Unter den Substituenten $R^1$ sind die im folgenden aufgeführten besonders bevorzugt:

Wasserstoff, Methyl, Äthyl, n-Propyl, n-Butyl, n-Pentyl, n-Hexyl, n-Heptyl, 2-Propyl, 2-Butyl, 2-Pentyl, 3-Hexyl, 2-Methylpropoxy, 2-Methylbutyl, 4,4-Dimethylpentyl, 5,5-Dimethylhexyl, Cyclopentyl, Cyclohexyl, Cycloheptyl.

Unter den Substituenten $R^2$ sind die im folgenden aufgeführten besonders bevorzugt:

n-Propyl, 2-Propyl, n-Butyl, 2-Butyl, t-Butyl, n-Pentyl, 3-Pentyl, 2-Methylbutyl, Neopentyl, n-Hexyl, 2-Äthylbutyl, 2,2-Dimethylbutyl, n-Heptyl, 1,1-Dimethyl-2-äthoxy-äthyl,

1,1-Dimethyl-2-methoxy-äthyl, Cyclopentyl, Cyclohexyl, Cycloheptyl.

Gegenstand der Erfindung ist ferner ein Verfahren zur Herstellung von Verbindungen der Formel I, daß dadurch gekennzeichnet ist, daß man

a) einen Aldehyd der Formel II

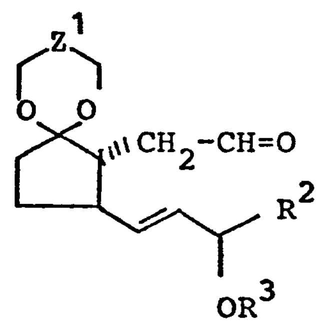

II

worin $R^2$ die zur Formel I angegebene Bedeutung hat, $R^3$ eine leicht abspaltbare Schutzgruppe bedeutet und worin $Z^1$ eine $-CH_2$-Gruppe, eine $-C(CH_3)_2$-Gruppe oder eine Einfachbindung bedeutet, mit einem Grignard-Reagenz der Formel III

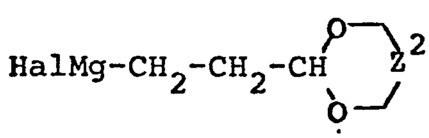

III

worin $Z^2$ eine $-CH_2$-Gruppe, eine $-C(CH_3)_2$-Gruppe oder eine Einfachbindung und Hal Chlor, Brom oder Jod bedeuten umsetzt zu einem Alkohol der Formel IV

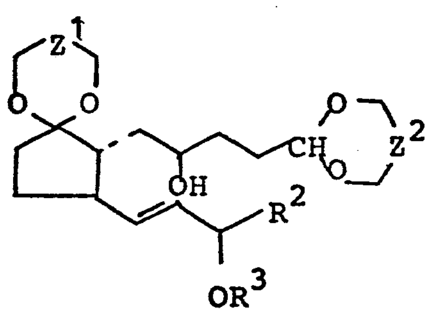

IV

worin $R^2$, $R^3$ und $Z^1$ die zur Formel II und $Z^2$ die zur Formel III angegebene Bedeutung haben,

b) in dem erhaltenen Alkohol der Formel IV durch saure Hydrolyse alle drei Schutzgruppen verseift, wobei ein Aldehyd der Formel V entsteht,

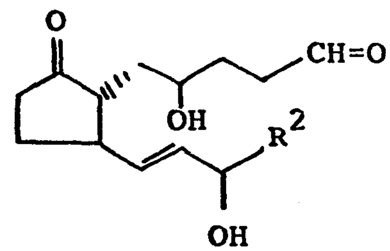

V

worin $R^2$ die in Formel I angegebene Bedeutung hat,

c) den erhaltenen Aldehyd der Formel V mit dem Ylid der Formel VI

$$(R^4)_3P{=}CH{-}COOCH_3 \qquad VI$$

worin die Reste $R^4$ gleich oder verschieden sind und geradkettiges $(C_1-C_4)$ Alkyl oder Phenyl bedeuten, umsetzt zu einer Verbindung der Formel VII

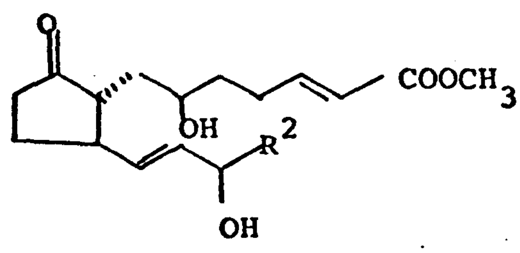

VII

worin $R^2$ die in Formel I angegebene Bedeutung hat,

d) den erhaltenen Ketoester der Formel VII durch Hydrolyse überführt in eine Verbindung der Formel I, worin $R^1$ Wasserstoff und $X^1$ und $Y^1$ zusammen Sauerstoff bedeuten und $X^2$ und $Y^2$ voneinander verschieden sind und jeweils Wasserstoff oder eine Hydroxylgruppe bedeuten und

$R^2$ die zur Formel I in Anspruch 1 genannte Bedeutung hat,

e) die erhaltene Verbindung mit einem komplexen Metallhydrid reduziert zu einer Verbindung der Formel I, worin $X^1$ und $Y^1$ sowie $X^2$ und $Y^2$ jeweils voneinander verschieden sind und jeweils Wasserstoff oder eine Hydroxylgruppe bedeuten, $R^1$ Wasserstoff bedeutet und $R^2$ die zur Formel I in Anspruch 1 genannte Bedeutung hat, oder

d') den erhaltenen Ketoester der Formel VII mit einem komplexen Metallhydrid reduziert und

e') die erhaltene Verbindung gemäß d) reduziert, oder

f) den Alkohol der Formel IV durch Oxydation in eine Verbindung der Formel VIII überführt,

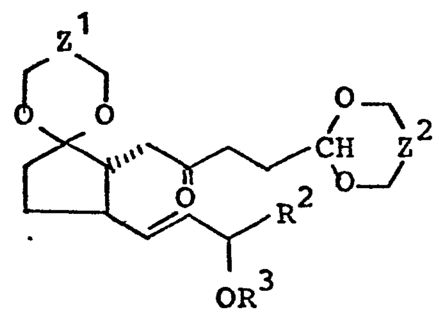

VIII

worin $R^2$ und $R^3$, sowie $Z^1$ und $Z^2$ die zur Formel II angegebene Bedeutung haben,

g) in der erhaltenen Ketoverbindung der Formel VIII durch saure Hydrolyse alle drei Schutzgruppen abspaltet, wobei eine Verbindung der Formel IX

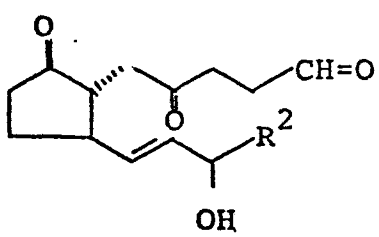

IX

worin $R^2$ die in Formel I angegebene Bedeutung hat, entsteht

h) den erhaltenen Keto-Aldehyd der Formel IX gemäß Verfahrensschritt c) mit dem Ylid der Formel VI umsetzt zu einer Verbindung der Formel X

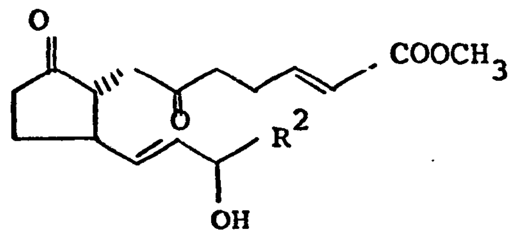

X

worin $R^2$ die zur Formel I angegebene Bedeutung hat,

i) den erhaltenen Ketoester der Formel X durch Hydrolyse überführt in eine Verbindung der Formel I, worin $R^2$ die zur Formel I genannte Bedeutung hat und

$R^1$ Wasserstoff und

$X^1$ und $Y^1$ sowie $X^2$ und $Y^2$ jeweils zusammen Sauerstoff darstellen,

j) gegebenenfalls eine Verbindung der Formel I, worin $X^1$, $X^2$, $Y^1$, $Y^2$ sowie $R^2$ die zur Formel I in Anspruch 1 angegegebene Bedeutung haben und $R^1$ Wasserstoff bedeutet, zu einer Verbindung der Formel I, worin $X^1$, $X^2$, $Y^1$, $Y^2$ sowie $R^2$ die zur Formel I in Anspruch 1 angegebenen Bedeutungen haben, wobei $R^1$ jedoch nicht Wasserstoff bedeutet, verestert,

k) und gegebenenfalls eine Verbindung der Formel I, worin $X^1$, $X^2$, $Y^1$, $Y^2$ sowie $R^2$ die zur Formel I in Anspruch 1 angegebenen Bedeutungen haben und $R^1$ Wasserstoff bedeutet, in ein physiologisch verträgliches Metall oder Aminsalz überführt.

Der Aldehyd der Formel V liegt vor im Gleichgewicht mit seinem inneren Halbacetal V a

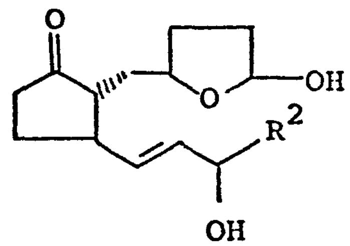

V a

worin $R^2$ die in Formel I angegebene Bedeutung hat.

Die Verfahrensschritte zur Herstellung von Verbindungen der Formel I ($R^1$ = H) können durchgeführt werden in Analogie zu dem in der DE-OS 2 546 313 (HOE 75/F 270) beschriebenen Verfahren. Die Veresterung von Verbindungen der Formel I ($R^1$ =H) erfolgt nach Methoden, wie sie in der DE-OS 26 28 564 (HOE 76/F 149) beschrieben sind.

Das Grignard-Reagenz der Formel III kann analog zu S. Büchi, H. Wüst, J. Org. Chem. 34, 1121 (1969) dargestellt werden.

Die erfindungsgemäßen Verbindungen der Formel I werden gewöhnlich in Form ihrer Racemate erhalten. Diese können gegebenenfalls nach den üblichen Methoden der Racemattrennung in Form der optisch aktiven Antipoden erhalten werden.

Die Ausgangsverbindungen der Formel IIkönnen gemäß der DE-OS 2 357 953 (HOE 73/F 355) hergestellt werden.

Außer den in den Beispielen genannten lassen sich insbesondere auch die folgenden Verbindungen herstellen:

6,15-Dihydroxy-9-oxo-16,16-dimethyl-18-oxa-(E)-2, (E)-13-prostadiensäure

6,9,15-Trihydroxy-16,16-dimethyl-(E)-2, (E)-13-prostadiensäure

15-Hydroxy-6,9-dioxo-16,16-dimethyl-18-oxa-(E)-2, (E)-13-prostadiensäure

15-Hydroxy-6,9-dioxo-16,16-dimethyl-18-oxa-(E)-2, (E)-13-prostadiensäure-methylester

6,15-Dihydroxy-9-oxo-20-homo-(E)-2, (E)-13-prostadiensäure

6,15-Dihydroxy-9-oxo-20-nor-(E)-2, (E)-13-prostadiensäure

6,9,15-Trihydroxy-20-homo-(E)-2, (E)-13-prostadiensäure

6,9,15-Trihydroxy-20-nor-(E)-2, (E)-13-prostadiensäure

15-Hydroxy-6,9-dioxo-20-homo-(E)-2, (E)-13-prostadiensäure

15-Hydroxy-6,9-dioxo-20-nor-(E)-2, (E)-13-prostadiensäure

6,9,15-Trihydroxy-20-homo-(E)-2, (E)-13-prostadiensäure-äthylester

6,9,15-Trihydroxy-20-nor-(E)-2, (E)-13-prostadiensäure-äthylester

15-Cyclohexyl-6,15-dihydroxy-9-oxo-16,17,18,19,20-pentanor-(E)-2, (E)-13-prostadiensäure

15-Cyclohexyl-6,9,15-trihydroxy-16,17,18,19,20-pentanor-(E)-2, (E)-13-prostadiensäure

15-Cyclohexyl-15-hydroxy-6,9-dioxo-16,17,18,19,20-pentanor-(E)-2, (E)-13-prostadiensäure

15-Cyclohexyl-15-hydroxy-6,9-dioxo-16,17,18,19,20-pentanor-(E)-2, (E)-13-prostadiensäure-methylester

Die erfindungsgemäßen Verbindungen zeichnen sich einerseits durch spasmogene, andererseits durch Blutplättchenaggregationshemmende, blutdrucksenkende, coronardilatierende und magensaftsekretionshemmende Eigenschaften aus. Verglichen mit den natürlichen Prostaglandinen sind sie stärker und länger anhaltend wirksam. Sie können daher als Arzneimittel angewandt werden.

Die erfindungsgemäßen Verbindungen der Formel I können als freie Säuren, in Form ihrer physiologisch unbedenklichen anorganischen und organischen Salze oder als Ester zur Anwendung kommen.

Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertige Alkoholen wie z.B. Äthanol, Äthylenglykol oder Glycerin, Ölen wie z.B. Sonnenblumenöl oder Lebertran, Äthern wie z.B. Diätyhylenglykoldimethyläther oder auch Polyäthern wie z.B. Polyäthylenglykole der auch in Gegenwart anderer pharmakologisch unbedenklicher Polymerträger wie z.B. Polyvinylpyrrolidon zur Anwendung gelangen.

Als Zubereitungen können die üblichen galenischen Infusionsder Injektionslösungen und Tabletten, sowie örtlich anwendbare Zubereitungen wie Cremes, Emulsionen, Suppositorien, insbesondere auch Aerosole in Frage kommen.

Eine weitere Anwendung der neuen Verbindungen liegt in der Kombination mit anderen Wirkstoffen, Neben anderen geeigneten Substanzen gehören dazu vor allem:

Sekretolytica wie z.B. Disolvon, ß-Sympathomimetica wie z.B. Salbutamol oder Aludrin oder Antitussiva wie z.B. Codein, fertilitätsregulierende Hormone bzw. Releasing-Hormone wie LH- FSH, Östradiol, LH-RH, Diuretika, wie z.B. Furosemid, Antidiabetika, wie z.B. Glycodiazin, Tolbutamid, Glibenclamid, Phenformin, Buformin, Metformin, Kreislaufmittel im weitesten Sinne, z.B. Coronardilatatoren wie Chromonar oder Prenylamin, blutdrucksenkende Stoffe wie Reserpin, α-Methyl-Dopa oder Clonidine oder Antiarrhythmika, Lipidsenker, Geriatrika und andere stoffwechselwirksame Präparate, Psychopharmaka, wie z.B. Chlordiazepoxid, Diazepam oder Meprobamat sowie Vitamine, oder Prostaglandine oder prostaglandin-ähnliche Verbindungen sowie auch Prostaglandinantagonisten und Prostaglanin-Bioxynthesehemmer, wie z.B. nicht-steroidale Antiphlogistika.

Die erfindungsgemäßen Verbindungen der Formel I zeigen blutplättchenaggregationshemmende und blutdrucksenkende Wirkung, wie durch Arachidonsäure-induzierte Blutplättchenaggregation in vitro bzw. durch intravenöse Verabreichung am wachen Hund festgestellt, bei einer Dosis von 0,01 bis 1 mg/kg. Die Einheitsdosis zur Behandlung des Menschen liegt daher in einem Bereich von 0,01 bis 1 mg/kg, bevorzugt zwischen 0,05 und 0,5 mg/kg. Die Tagesdosis liegt zwischen 0,03 und 3 mg/kg, bevorzugt zwischen 0,15 und 1,5 mg/kg.

Die Verbindungen der Formel IV, V, VII, VIII, IX, X sind neue wertvolle Zwischenprodukte für die Herstellung von Verbindungen der Formel I.

**Beispiel 1:**

**1-Brom-2-(1,3-dioxo-2-cyclohexyl)-äthan**

In 851 g 1,3-Propandiol werden bei - 5° 350 g Bromwasserstoff eingeleitet. Dann wurde unter Kühlung bei max. 1o°C 165 g = 195 ml Acrolein zugetropft. Es wurde 1 Stunde bei Raumtemperatur nachgerührt, 4 x mit n-Hexan ausgerührt. Die org. Phase abgetrennt, zwei Stunden mit festem $NaHCO_3$ gerührt, abgesaugt, das Lösungsmittel wurde i.V. entfernt.

Rohausbeute: 408 g

Über eine 10 cm Kolonne wurde fraktioniert.

Ausbeute: 373 g farblose Flüssigkeit $Sdp_{15\ mm}$ = 89 - 91°C

NMR: $\delta$ 1,1 - 2,4 (m,4) $-CH_2-$, $-CH_2-CH\langle$;

3,3 - 4,3 (m,6) $-\underline{CH_2}-Br$, $-\underline{CH_2}-O-$;

4,6 (t,1) -O-CH-O

**Beispiel 2:**

**7-(3-Tetrahydropyranyloxy-(E)-1-octen-1-yl)-1,4-dioxa-spiro[4,4]-non-6-yl-acetaldehyd II**

4,2 g 6[(1,3-Dithia-2-cyclopentyl)-methyl]-7-(3-tetra-hydropyranyloxy-(E)-1-octen-1-yl)-1,4-dioxaspiro[4,4]-nonan (dargestellt analog wie in Beispiel 9 a HOE 73 F 355 DOS beschrieben) wurden in 25 ml Dimethylformamid mit 3,7 ml Methyljodid, 0,8 g Calciumcarbonat und 1,7 ml $H_2O$ 2 Stunden auf 50° erwärmt, vom Calciumcarbonat abfiltriert, der Niederschlag mit Aceton gewaschen, die organsiche Phase eingeengt und in Diäthyläther gelöst. Nach Waschen mit Wasser und Trocknen über $Na_2SO_4$ wurde der Äther abdestilliert.

Es waren 3,4 g eines bräunlichen Öles entstanden.
U.R. Aldehydbande bei 1740 $cm^{-1}$.

**Beispiel 3:**

**6[(3-Hydroxy-1(1,3-dioxa-2-cyclohexyl)-butyl]-7(3-tetrahydro-pyranyloxy-1-ootenyl)-1,4-dioxa-spiro[4,4]nonan IV**

1,0 g Magnesium werden mit Jodkristallen angeätzt, mit 10 ml abs. THF versetzt und 6 g 1-Brom-2(1,3-dioxo-2-cyclohexyl)-äthan (Beispiel 1) in 20 ml abs. THF innerhalb 20 Minuten zugetropft, (Reaktion springt beim leichten Erwärmen an) dann wird 2 Stdn. bei 50°C weitergerührt und 3,4 g 7(3-Tetrahydropyranyloxy-(E)-1-octen-1-yl)-1,4-dioxaspiro[4,4]-non-6-yl-acetaldehyd (Beispiel 2) in 20 ml abs. Diäthyläther zugetropft. Man rührt 3 Stdn. bei Raumtemperatur weiter. Es wird mit Eis und verdünnter Salzsäure versetzt, dann mit NaCl gesättigt und mit Äther extrahiert. Die organische Phase wird 2 x mit NaCl/Bicarbonat-Lösung gewaschen mit $MgSO_4$ getrocknet, i. Vak. eingeengt.

Rohausbeute: 3,4 g

Über eine Kieselgelsäule mit Cyclohexan/Essigester 1 : 1, dann 2 : 1, dann 0 : 1 als Elutionsmittel wurden Verunreinigungen abgetrennt

Ausbeute: 2,6 g leicht gelbes Öl

Dünnschichtchromatogramm: Essigester : Eisessig = 9 : 1

$R_f$ = 0,50

**Beispiel 4:**

**1[4-(1-Formyl-3-hydroxy)-butyl]-2(3-hydroxy-1-octenyl)-cyclopentan-5-on V**

230 mg 6[(3-Hydroxy-1(1,3-dioxa-2-cyclohexyl)butyl]-7(3-tetrahydropyranyloxy-1-octenyl)-1,4-dioxaspiro[4,4]-nonan (Beispiel 3) 5 ml Dimethylglykol, 5 ml Wasser und 1 ml gesättigte Oxalsäurelösung werden 6 Stunden bei 50°C - 55° gerührt. Es wird abgekühlt, mit 10 ml gesättigter Natriumchlorid-Lösung versetzt, mit NaCl gesättigt, mit Essigester extrahiert.

Die org. Phase wird 1 x mit ges. NaCl-Lösung/Bicarbonatlösung gewaschen über $MgSO_4$ getrocknet, i. Vak. eingeengt. Über eine Säule aus Kieselgel mit anfangs Äther/Essigester 1 : 1 als Elutionsmittel später mit Essigester/Eisessig 99 : 1 wird von Verunreinigungen abgetrennt.

Ausbeute: 210 mg leicht gelbes Öl

$R_f$ Wert: 0,52 Essigester : Eisessig = 9 : 1

NMR: $\delta$ 0,8 - 2,7 (m, 21) $-CH_2-$, -CH-;
3,5 - 4,6 (m, 7) -CH-O, OH
5,4 - 5,7 (m, 2) olefinische Protonen

Die Verbindung V liegt als inneres Halbacetal V a vor.

0008076

**Beispiel 5:**

**6,15-Dihydroxy-9-oxo-(E)-2, (E)-13-prostadiensäure-methylester VII**

110 mg 1[4(1-Formyl-3-hydroxy)-butyl]-2(3-hydroxy-1-octenyl)-cyclopentan-5-on (Beispiel 4) sowie 290 mg Carbomethoxymethylen-triphenyl-phosphoran VI (Lit: v. Fasciulus Helv. chim. acta XL, S. 1247 (1957)) werden in 10 ml abs. Benzol unter $N_2$ 5 Stunden bei Raumtemperatur gerührt und 2 Tage bei - 10°C aufbewahrt. Es wird eingeengt, der Rückstand über eine Säule aus Kieselgel gereinigt. (Elutionsmittel, Toluol : Essigester 1 : 1)

Ausbeute: 43 mg hellgelbes Öl

$R_f$: 0,64 Essigester: Eisessig = 9 : 1

**Beispiel 6:**

**6,15-Dihydroxy-9-oxo-(E)-2, (E)-13-prostadiensäure I**

280 mg 6,15-Dihydroxy-9-oxo-(E)-2, (E)-13-prostadiensäure-methylester VII (Beispiel 5) werden in 10 ml Methanol gelöst und mit der 3 Äquivalenten 0,5 N Natronlauge versetzt. Man rührt bei Raumtemperatur 8 Stunden und verfolgt den Reaktionsverlauf im Dünnschichtchromatogramm. Nach Beendigung der Verseifung wird das Lösungsmittel vorsichtig im Vakuum entfernt, der Rückstand mit Essigester/Wasser versetzt, mit 1 N Citronensäure auf pH 4-5 angesäuert. Die org. Phase abgetrennt, mit $MgSO_4$ getrocknet, i. Vak. eingeengt.

Ausbeute: 270 mg gelbbraunes Öl, welches durch Chromatographie an einer Kieselgelsäule mit Essigsäureäthylester/Eisessig 95 : 5 gereinigt wird.

Reinausbeute: 160 mg hellgelbes Öl

**Beispiel 7:**

**6[(3-Oxo-1(1,3-dioxa-2-cyclohexyl)butyl]-7(3-tetrahydro-pyranyloxy-1-octenyl)-1,4-dioxaspiro[4,4]nonan VIII**

0,5 g 6[(3-Hydroxy-1(1,3-dioxa-2-cyclohexyl)butyl]-7(3-tetrahydro-pyranyloxy-1-octenyl)-1,4-dioxaspiro[4,4]-nonan (Beispiel 3) werden in 30 ml Aceton gelöst. Bei - 20 bis -25°C werden unter Argon 2 ml Iones Reagenz (2,1 g Chromsäure, 6 ml Wasser, 1,7 ml konz. Schwefelsäure) zugetropft. Man rührt 30 Minuten, gibt man dann 3 ml Isopropanol zu und rührt weitere 10 Minuten, um überschüssiges Oxydationsreagenz zu zerstören. Es wurde dann mit 100 ml Methylenchlorid und 100 ml Wasser versetzt, ausgeschüttelt, die Phasen getrennt, der organische Extrakt mit $MgSO_4$ getrocknet und das Lösungsmittel im Vakuum bei maximal + 5°C eingeengt. Die Ausbeute an der Verbindung betrug 0,38 g fast farbloses, klares Öl

Dünnschichtchromatogramm (Essigsäureäthylester - Essigsäure auf Kieselgelplatten-Merck = 97,5 : 2,5) $R_f$ = 0,85

**Beispiel 8:**

**1[4-(1-Formyl-3-oxo)-butyl]-2(3-hydroxy-1-octenyl)-cyclopentan-5-on IX**

265 mg 6[(3-Oxo-1(1,3-dioxa-2-cyclohexyl)butyl]-7(3-tetrahydro-pyranyloxy-1-octenyl)-1,4-dioxaspiro[4,4]nonan (Beispiel 7)

5 ml Dimethylglykol, 5 ml Wasser und 1 ml gesättigte Oxalsäurelösung werden 13 Stunden bei 50°C gerührt. Es wird abgekühlt, mit 10 ml gesättigter Natriumchlorid-Lösung versetzt, mit NaCl gesättigt, mit Essigester extrahiert.

Die org. Phase wird 1 x mit ges. NaCl-Lösung/Bicarbonatlösung gewaschen über $MgSO_4$ getrocknet, i. Vak. eingeengt. Über eine Säule aus Kieselgel mit anfangs Äther/Essigester 1 : 1 als Elutionsmittel später mit Essigester/Eisessig 99 : 1 wird von Verunreinigungen abgetrennt.

Ausbeute: 170 mg farbloses Öl

Dünnschichtchromatogramm (Essigsäureäthylester -Eisessig = 97,5 : 2,5) auf Kieselgelplatten - Merck

$R_f$ = 0,83

**Beispiel 9:**

**15-Hydroxy-6,9-dioxo-(E)-2, (E)-13-prostadiensäure-methylester X**

160 mg 1[4(1-Formyl-3-oxo)-butyl]-2(3-hydroxy-1-octenyl)-cyclopentan-5-on (Beispiel 8) sowie 240 mg Carbomethoxymethylen-triphenyl-phosphoran VI (Lit: v. Fasciculus Helv. chim. acta XL, S 1247 (1957)) werden in 15 ml abs. Benzol unter $N_2$ 16 Stunden bei Raumtemperatur gerührt und 2 Tage bei - 10°C aufbewahrt. Es wird eingeengt, der Rückstand über eine Säule aus Kieselgel gereinigt. (Elutionsmittel, Toluol : Essigester 1 : 1 )

Ausbeute: 150 mg helles Öl

Dünnschichtchromatogramm: Kieselgelplatte der Fa. Merck

2 Isomere

$R_f$ = 0,78

0,68

NMR: δ 0,8 - 2,5 (m,23) $-CH_2-$, $-CH-$,

3,0 - 3,5 (m,1) OH

3,5 - 4,4 (m,1) -C<u>H</u>-OH,

3,8 (s,3) $OCH_3$

5,5 - 6,3 (m,2) olefin. Prot.

6,5 - 7,2 (m,2) olefin. Prot.

**Beispiel 10:**

**15-Hydroxy-6,9-dioxo-(E)-2, (E)-13-prostadiensäure I**

140 mg 15-Hydroxy-6,9-dioxo-(E)-2, (E)-13-prostadiensäure-methylester (Beispiel 9)
werden in 15 ml Methanol gelöst und mit der 2 Äquivalenten 0,5 N Natronlauge versetzt. Man rührt bei Raumtemperatur 8 Stunden und verfolgt den Reaktionsverlauf im Dünnschichtchromatogramm. Nach Beendigung der Verseifung wird das Lösungsmittel vorsichtig im Vakuum entfernt, der Rückstand mit Essigester/Wasser versetzt, mit 1 N Citronensäure auf pH 4 - 5 angesäuert. Die org. Phase abgetrennt, mit $MgSO_4$ getrocknet, i. Vak. eingeengt.

Ausbeute: 130 mg gelbbraunes Öl, welches durch Chromatographie an einer Kieselgelsäule mit Essigsäureäthylester/Eisessig 95 : 5 gereinigt wird.

Reinausbeute: 80,0 mg helles Öl

NMR: $\delta$ 0,9 - 2,5 (m,23) $-CH_2-$, -CH-
3,5 - 4,3 (m,1) -C<u>H</u>-OH
4,8 - 5,4 (breites Signal,2) O<u>H</u>, COOH
5,3 - 6,2 (m,2) olef. Protonen
6,6 - 7,1 (m,2) olef. Protonen

**Beispiel 11:**

**6,9,15-Trihydroxy-(E)-2, (E)-13-prostadiensäure-methylesterI**

126 mg Natriumborhydrid wurden in 1,0 ml $H_2O$ und 4 ml Methanol gelöst, auf 0°C abgekühlt und 252 mg 6,15-Dihydroxy-9-oxo-(E)-2, (E)-13-prostadiensäure-methylester (Beispiel 5) in 5 ml Methanol gelöst zugetropft, 5 h bei 0°C und 2 Stunden bei Raumtemperatur gerührt. Die Reaktionsmischung wurde mit Eisessig neutralisiert, das Lösungsmittel im Vakuum abdestilliert, der Rückstand in Äther aufgenommen, mit Wasser gewaschen getrocknet und eingeengt.

Das Rohprodukt wurde in Cyclohexan/Essigester 95 : 5 über eine Kieselgelsäule, 14 cm Höhe und 2 cm Durchmesser, filtriert und eingeengt.

Es wurden 210 mg farbloses Öl erhalten

$R_f$ = 0,72 (Kieselgel nach Merck Essigester/Eisessig 97,5 : 2,5

U.R. = keine Ketonbande bei 1695 $cm^{-1}$

**Beispiel 12:**

**6,9,15-Trihydroxy-(E)-2, (E)-13-prostadiensäure I**

100 mg 6,9,15-Trihydroxy-(E)-2, (E)-13-prostadiensäure-methylester (Beispiel 11) werden analog Beispiel 10 umgesetzt.

Man erhält 60 mg hellgelbes Öl

Dünnschichtchromatogramm: 2 Isomere Essigsäure : Eisessig = 97,5 : 2,5 $R_f = 0,45$

$R_f = 0,38$

0008076

**Beispiel 13:**

**6,15-Dihydroxy-9-oxo-(E)-2, (E)-13-prostadiensäure-äthylester I**

Zu 80 mg 6,15-Dihydroxy-9-oxo-(E)-2, (E)-13-prostadiensäure (Beispiel 6) in 6 ml Äther wird unter Eiskühlung mit 5 ml einer 1 M Diazoäthanlösung in Äther versetzt. Man läßt noch 30 Min. rühren und verdampft das Lösungsmittel mit dem überschüssigen Diazoäthan im Vakuum. Das Produkt ist chromatographisch rein.

Ausbeute: 85 mg

NMR: analog Beispiel 6

zusätzliche Ester: δ 1,25 (t,3) $-COOCH_2CH_3$;

Protonen 4,25 (q,2) $-COO\underline{CH_2}CH_3$

**Beispiel 14:**

**15-Hydroxy-6,9-dioxo-(E)-2, (E)-13-prostadiensäureäthylester I**

Eine Lösung von 40 mg 15-Hydroxy-6,9-dioxo-(E)-2, (E)-13-prostadiensäure in 10 ml Äther wird unter Eiskühlung mit 5 ml einer 1 M Diazoäthanlösung in Äther versetzt. Man läßt noch 30 Min. rühren und verdampft das Lösungsmittel mit dem überschüssigen Diazoäthan im Vakuum. Das Produkt ist chromatographisch rein.

Ausbeute: 43 mg

NMR: analog Beispiel 10

zusätzliche Ester: δ 1,24 (t,3) $-COOCH_2\underline{CH_3}$,

4,25 (q,2) $-COO\underline{CH_2}CH_3$

**Beispiel 15:**

**6,9,15-Trihydroxy-(E)-2, (E)-13-prostadiensäure-Thromethaminsalz I**

Eine Lösung von 71 mg 6,9,15-Trihydroxy-(E)-2, (E)-13-prostadiensäure (Beispiel 12) in 4 ml Äthanol wird mit einer äthanolischen Lösung von 24,2 g Thromethaminbase versetzt und das Lösungsmittel abgedampft zuletzt im Hochvakuum.

Ausbeute: 95 mg Thromethaminsalz I als Kristalliner Rückstand.

**Beispiel 16:**

**15-Hydroxy-6,9-dioxo-(E)-2, (E)-13-prostadiensäure-Thrometh-salz I**

Eine Lösung von 35,4 mg 15-Hydroxy-6,9-dioxo-(E)-2, (E)-13-prostadiensäure (Beispiel 14) in 3 ml Äthanol wird mit einer äthanolischen Lösung von 12,1 mg Thromethaminbase versetzt und das Lösungsmittel abgedampft. zuletzt im Hochvakuum.

Ausbeute: 47 mg Thromethaminsalz I als öligen Rückstand

PATENTANSPRÜCHE:

1. Verbindungen der Formel I

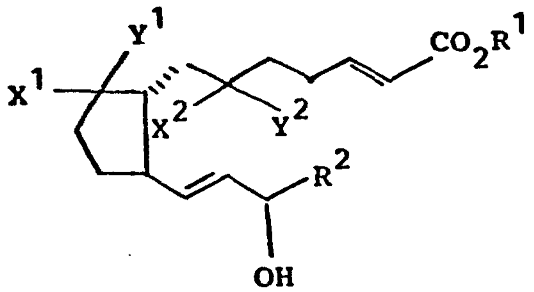

I

in welcher bedeuten

$X^1$ und $Y^1$ sowie $X^2$ und $Y^2$ zusammen Sauerstoff oder jeweils Wasserstoff oder eine Hydroxylgruppe, wobei $X^1$ und $Y^1$ sowie $X^2$ und $Y^2$ verschieden sind

$R^1$ a) Wasserstoff oder einen geradkettigen, verzweigten gesättigten oder ungesättigten aliphatischen oder cycloaliphatischen Kohlenwasserstoffrest mit bis zu zehn Kohlenstoffatomen oder

b) ein physiologisch verträgliches Metall- oder $NH_4^{\pm}$ Ion oder ein Ammoniumion, das sich von einem primären, sekundären oder tertiären Amin ableitet,

$R^2$ einen geraden, verzweigten, gesättigten oder ungesättigten aliphatischen Kohlenwasserstoffrest mit zwei bis sieben Kohlenstoffatomen, in dem eine nicht endständige $CH_2$-Gruppe durch ein Sauerstoffatom ersetzt sein kann, oder einen Cycloalkylrest mit drei bis sieben Kohlenstoffatomen.

2. Verbindungen der Formel IV

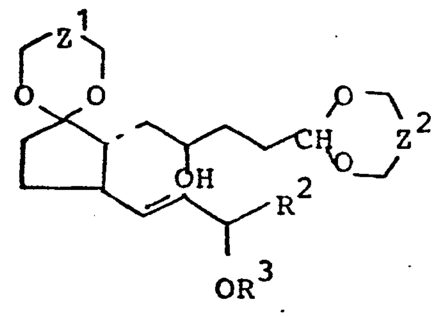

IV

worin $R^2$, $R^3$ und $Z^1$ die zur Formel II und $Z^2$ die zur Formel III angegebene Bedeutung haben.

3. Verbindungen der Formel V

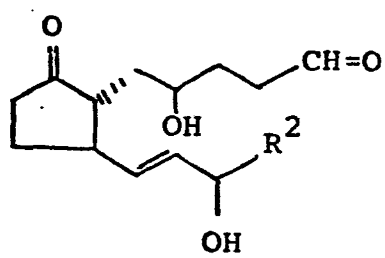

V

worin $R^2$ die in Formel I angegebene Bedeutung hat.

4. Verbindungen der Formel VII

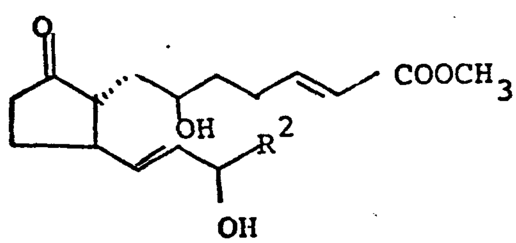

VII

worin $R^2$ die in Formel I angegebene Bedeutung hat.

5. Verbindungen der Formel VIII

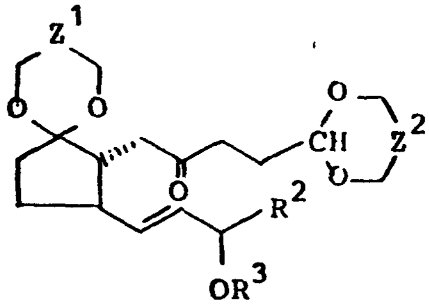

VIII

worin $R^2$ und $R^3$ sowie $Z^1$ und $Z^2$ die zur Formel II angegebene Bedeutung haben.

6. Verbindungen der Formel IX

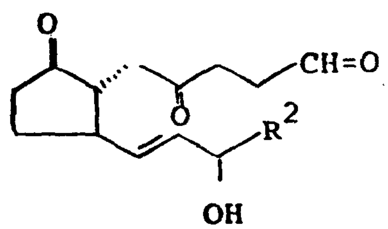

IX

worin $R^2$ die in Formel I angegebene Bedeutung hat.

7. Verbindungen der Formel X

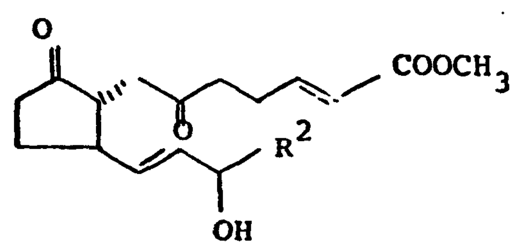

X

worin $R^2$ die zur Formel I angegebene Bedeutung hat.

8. Verfahren zur Herstellung von Verbindungen der Formel I

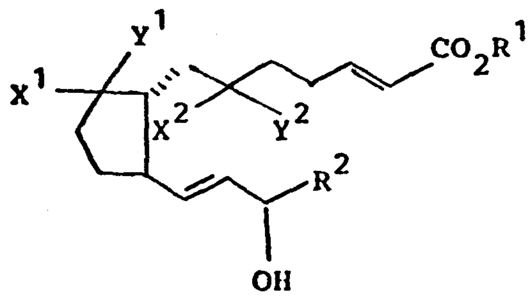

I

dadurch gekennzeichnet, dass man eine Verbindung der Formel Ia

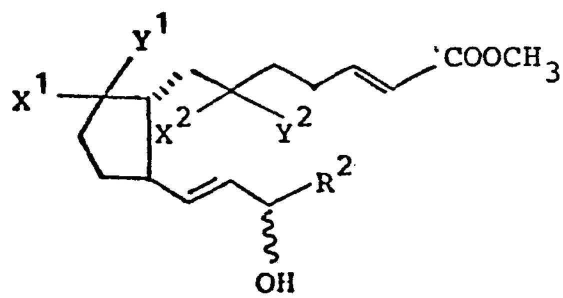

Ia

worin $R^2$, $X^1$, $Y^1$, $X^2$ und $Y^2$ die zur Formel I in Anspruch 1 genannten Bedeutungen haben, durch Hydrolyse überführt in eine Verbindung der Formel I, worin $R^1$ Wasserstoff bedeutet, gegebenenfalls eine Verbindung der Formel I, worin $X^1$, $X^2$, $Y^1$, $Y^2$ sowie $R^2$ die zur Formel I in Anspruch 1 angegebene Bedeutung haben und $R^1$ Wasserstoff bedeutet, zu einer Verbindung der Formel I, worin $X^1$, $X^2$, $Y^1$, $Y^2$ sowie $R^2$ die zur Formel I in Anspruch 1 angegebenen Bedeutungen haben, wobei $R^1$ jedoch nicht Wasserstoff bedeutet, verestert, und gegebenenfalls eine Verbindung der Formel I, worin $X^1$, $X^2$, $Y^1$, $Y^2$ sowie $R^2$ die zur Formel I in Anspruch 1 angegebenen Bedeutungen haben und $R^1$ Wasserstoff bedeutet, in ein physiologisch verträgliches Metall oder Aminsalz überführt.

9. Verfahren zur Herstellung von Verbindungen der Formel I, dadurch gekennzeichnet, daß man

a) einen Aldehyd der Formel II

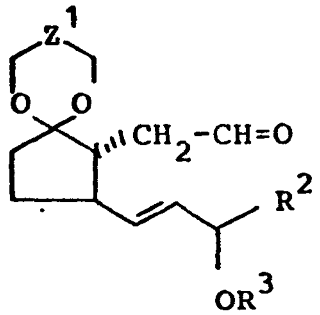

II

worin $R^2$ die zur Formel I angegebene Bedeutung hat, $R^3$ eine leicht abspaltbare Schutzgruppe bedeutet und worin $Z^1$ eine $-CH_2$-Gruppe, eine $-C(CH_3)_2$-Gruppe oder eine Einfachbindung bedeutet, mit einem Grignard-Reagenz der Formel III

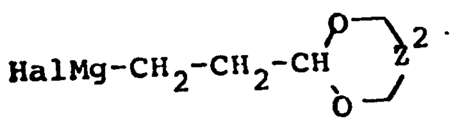

III

worin $Z^2$ eine $-CH_2$-Gruppe, eine $-C(CH_3)_2$-Gruppe oder eine Einfachbindung und Hal Chlor, Brom oder Jod bedeuten umsetzt zu einem Alkohol der Formel IV

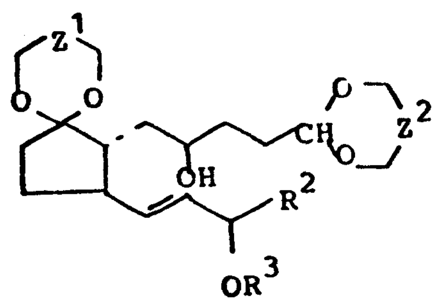

IV

worin $R^2$, $R^3$ und $Z^1$ die zur Formel II und $R^2$ die zur Formel III angegebene Bedeutung haben,

b) in dem erhaltenen Alkohol der Formel IV durch saure Hydrolyse alle drei Schutzgruppen verseift, wobei ein Aldehyd der Formel V entsteht,

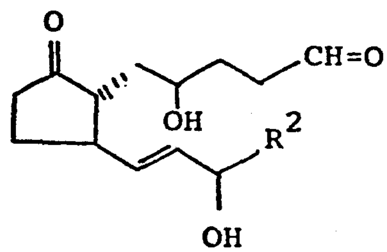

V

worin $R^2$ die in Formel I angegebene Bedeutung hat,

c) den erhaltenen Aldehyd der Formel V mit dem Ylid der Formel VI

$(R^4)_3P=CH-COOCH_3$ VI

worin die Reste $R^4$ gleich oder verschieden sind und geradkettiges $(C_1-C_4)$Alkyl oder Phenyl bedeuten, umsetzt zu einer Verbindung der Formel VII

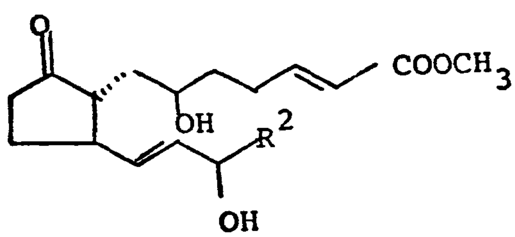

VII

worin $R^2$ die in Formel I angegebene Bedeutung hat,

d) den erhaltenen Ketoester der Formel VII durch Hydrolyse überführt in eine Verbindung der Formel I, worin $R^1$ Wasserstoff und $X^1$ und $Y^1$ zusammen Sauerstoff bedeuten und $X^2$ und $Y^2$ voneinander verschieden sind und jeweils Wasserstoff oder eine Hydroxylgruppe bedeuten und $R^2$ die zur Formel I in Anspruch 1 genannte Bedeutung hat,

e) die erhaltene Verbindung mit einem komplexen Metallhydrid reduziert zu einer Verbindung der Formel I, worin $X^1$ und $Y^1$ sowie $X^2$ und $Y^2$ jeweils voneinander verschieden sind und jeweils Wasserstoff oder eine Hydroxylgruppe bedeuten, $R^1$ Wasserstoff bedeutet und $R^2$ die zur Formel I in Anspruch 1 genannte Bedeutung hat, oder

d')den erhaltenen Ketoester der Formel VII mit einem komplexen Metallhydrid reduziert und

e')die erhaltene Verbindung gemäß d) reduziert, oder

f) den Alkohol der Formel IV durch Oxydation in eine Verbindung der Formel VIII überführt,

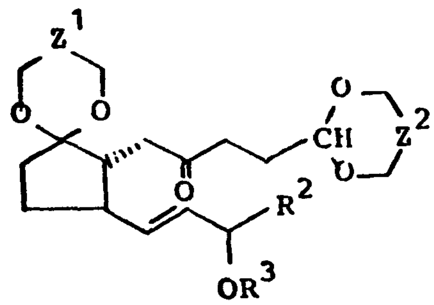

VIII

worin $R^2$ und $R^3$, sowie $Z^1$ und $Z^2$ die zur Formel II angegebene Bedeutung haben,

g) in der erhaltenen Ketoverbindung der Formel VIII durch saure Hydrolyse alle drei Schutzgruppen abspaltet, wobei eine Verbindung der Formel IX

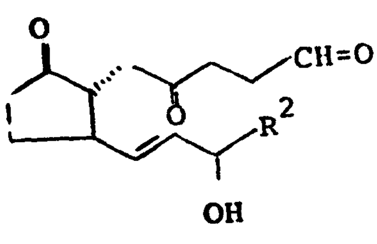

IX

worin $R^2$ die in Formel I angegebene Bedeutung hat, entsteht

h) den erhaltenen Keto-Aldehyd der Formel IX gemäß Verfahrensschritt c) mit dem Ylid der Formel VI umsetzt zu einer Verbindung der Formel X

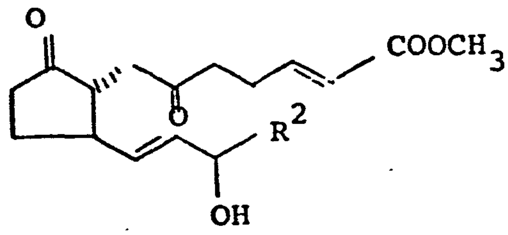

X

worin $R^2$ die zur Formel I angegebene Bedeutung hat,

i) den erhaltenen Ketoester der Formel X durch Hydrolyse überführt in eine Verbindung der Formel I, worin $R^2$ die zur Formel I genannte Bedeutung hat und

$R^1$ Wasserstoff und

$X^1$ und $Y^1$ sowie $X^2$ und $Y^2$ jeweils zusammen Sauerstoff darstellen,

j) gegebenenfalls eine Verbindung der Formel I, worin $X^1$, $X^2$, $Y^1$, $Y^2$ sowie $R^2$ die zur Formel I in Anspruch 1 angegebene Bedeutung haben und $R^1$ Wasserstoff bedeutet, zu einer Verbindung der Formel I, worin $X^1$, $X^2$, $Y^1$, $Y^2$ sowie $R^2$ die zur Formel I in Anspruch 1 angegebenen Bedeutungen haben, wobei $R^1$ jedoch nicht Wasserstoff bedeutet, verestert,

k) und gegebenenfalls eine Verbindung der Formel I, worin $X^1$, $X^2$, $Y^1$, $Y^2$ sowie $R^2$ die zur Formel I in Anspruch 1 angegebenen Bedeutungen haben und $R^1$ Wasserstoff bedeutet, in ein physiologisch verträgliches Metall oder Aminsalz überführt.

10. Verfahren zur Herstellung von Arzneimitteln, dadurch gekennzeichnet, dass man eine Verbindung der in Anspruch 1 genannten Formel I, gegebenenfalls mit üblichen pharmazeutischen Trägern und/oder Stabilisatoren, in eine therapeutisch geeignete Darreichungsform bringt.

11. Verbindungen der Formel I zur Verwendung als Arzneimittel.

Patentansprüche für Österreich:

1. Verfahren zur Herstellung von Verbindungen der Formel I

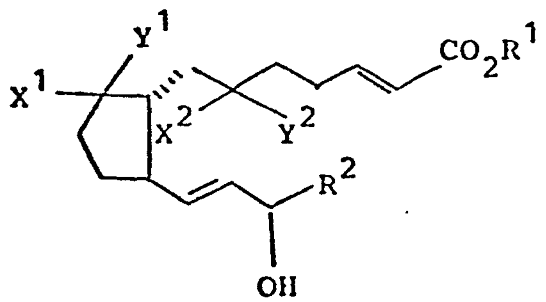

I

in welcher bedeuten

$X^1$ und $Y^1$ sowie $X^2$ und $Y^2$ zusammen Sauerstoff oder jeweils Wasserstoff oder eine Hydroxylgruppe, wobei $X^1$ und $Y^1$ sowie $X^2$ und $Y^2$ verschieden sind

$R^1$ a) Wasserstoff oder einen geradkettigen, verzweigten gesättigten oder ungesättigten aliphatischen oder cycloaliphatischen Kohlenwasserstoffrest mit bis zu zehn Kohlenstoffatomen oder

b) ein physiologisch verträgliches Metall- oder $NH_4^{\pm}$ Ion oder ein Ammoniumion, das sich von einem primären, sekundären oder tertiären Amin ableitet,

$R^2$ einen geraden, verzweigten, gesättigten oder ungesättigten aliphatischen Kohlenwasserstoffrest mit zwei bis sieben Kohlenstoffatomen, in dem eine nicht endständige $CH_2$-Gruppe durch ein Sauerstoffatom ersetzt sein kann, oder einen Cycloalkylrest mit drei bis sieben Kohlenstoffatomen,

dadurch gekennzeichnet, dass man eine Verbindung der Formel Ia

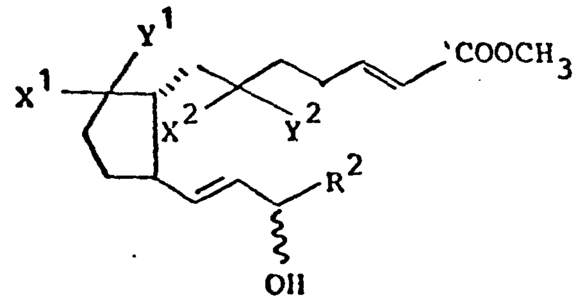

Ia

worin $R^2$, $X^1$, $Y^1$, $X^2$ und $Y^2$ die zur Formel I in Anspruch 1 genannten Bedeutungen haben, durch Hydrolyse überführt in eine Verbindung der Formel I, worin $R^1$ Wasserstoff bedeutet, gegebenenfalls eine Verbindung der Formel I, worin $X^1$, $X^2$, $Y^1$, $Y^2$ sowie $R^2$ die zur Formel I in Anspruch 1 angegebene Bedeutung haben und $R^1$ Wasserstoff bedeutet, zu einer Verbindung der Formel I, worin $X^1$, $X^2$, $Y^1$, $Y^2$ sowie $R^2$ die zur Formel I in Anspruch 1 angegebenen Bedeutungen haben, wobei $R^1$ jedoch nicht Wasserstoff bedeutet, verestert, und gegebenenfalls eine Verbindung der Formel I, worin $X^1$, $X^2$, $Y^1$, $Y^2$ sowie $R^2$ die zur Formel I in Anspruch 1 angegebenen Bedeutungen haben und $R^1$ Wasserstoff bedeutet, in ein physiologisch verträgliches Metall oder Aminsalz überführt.

2. Verfahren zur Herstellung von Verbindungen der Formel I, dadurch gekennzeichnet, daß man

a) einen Aldehyd der Formel II

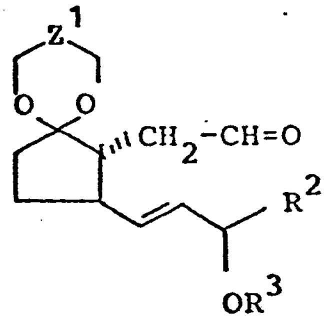

II

worin $R^2$ die zur Formel I angegebene Bedeutung hat, $R^3$ eine leicht abspaltbare Schutzgruppe bedeutet und worin $Z^1$ eine $-CH_2$-Gruppe, eine $-C(CH_3)_2$-Gruppe oder eine Einfachbindung bedeutet, mit einem Grignard-Reagenz der Formel III

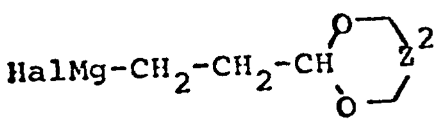

III

worin $Z^2$ eine $-CH_2$-Gruppe, eine $-C(CH_3)_2$-Gruppe oder eine Einfachbindung und Hal Chlor, Brom oder Jod bedeuten umsetzt zu einem Alkohol der Formel IV

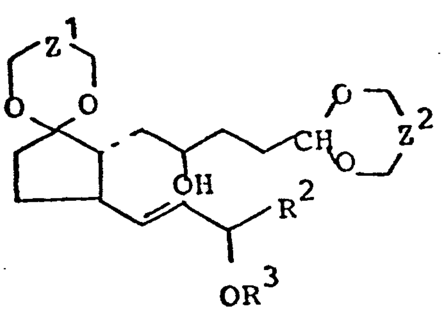

IV

worin $R^2$, $R^3$ und $Z^1$ die zur Formel II und $R^2$ die zur Formel III angegebene Bedeutung haben,

b) in dem erhaltenen Alkohol der Formel IV durch saure Hydrolyse alle drei Schutzgruppen verseift, wobei ein Aldehyd der Formel V entsteht,

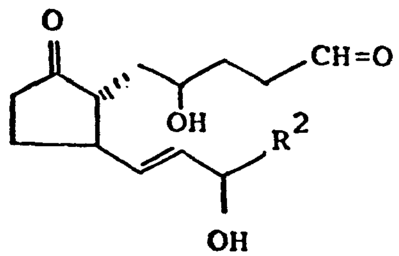

V

worin $R^2$ die in Formel I angegebene Bedeutung hat,

c) den erhaltenen Aldehyd der Formel V mit dem Ylid der Formel VI

$(R^4)_3P{=}CH{-}COOCH_3$ VI

worin die Reste $R^4$ gleich oder verschieden sind und geradkettiges $(C_1{-}C_4)$Alkyl oder Phenyl bedeuten, umsetzt zu einer Verbindung der Formel VII

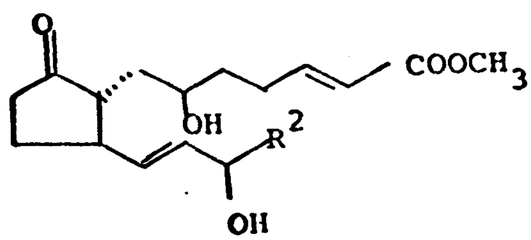

VII

worin $R^2$ die in Formel I angegebene Bedeutung hat,

d) den ~~erhaltenen~~ Ketoester der Formel VII durch Hydrolyse überführt in eine Verbindung der Formel I, worin $R^1$ Wasserstoff und $X^1$ und $Y^1$ zusammen Sauerstoff bedeuten und $X^2$ und $Y^2$ voneinander verschieden sind und jeweils Wasserstoff oder eine Hydroxylgruppe bedeuten und $R^2$ die zur Formel I in Anspruch 1 genannte Bedeutung hat,

e) die erhaltene Verbindung mit einem komplexen Metallhydrid reduziert zu einer Verbindung der Formel I, worin $X^1$ und $Y^1$ sowie $X^2$ und $Y^2$ jeweils voneinander verschieden sind und jeweils Wasserstoff oder eine Hydroxylgruppe bedeuten, $R^1$ Wasserstoff bedeutet und $R^2$ die zur Formel I in Anspruch 1 genannte Bedeutung hat, oder

d') den erhaltenen Ketoester der Formel VII mit einem komplexen Metallhydrid reduziert und

e') die erhaltene Verbindung gemäß d) reduziert, oder

f) den Alkohol der Formel IV durch Oxydation in eine Verbindung der Formel VIII überführt,

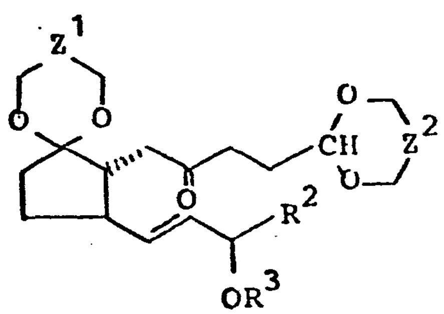

VIII

worin $R^2$ und $R^3$, sowie $Z^1$ und $Z^2$ die zur Formel II angegebene Bedeutung haben,

g) in der erhaltenen Ketoverbindung der Formel VIII durch saure Hydrolyse alle drei Schutzgruppen abspaltet, wobei eine Verbindung der Formel IX

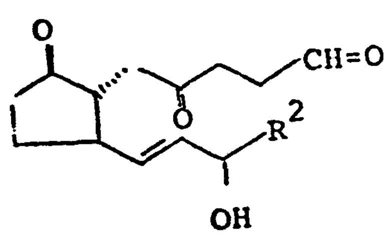

IX

worin $R^2$ die in Formel I angegebene Bedeutung hat, entsteht

h) den erhaltenen Keto-Aldehyd der Formel IX gemäß Verfahrensschritt c) mit dem Ylid der Formel VI umsetzt zu einer Verbindung der Formel X

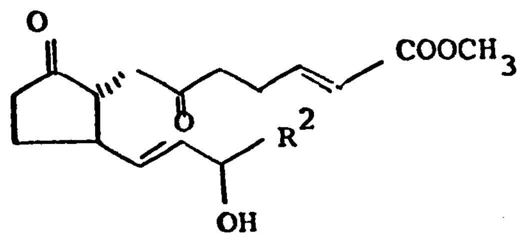

X

worin $R^2$ die zur Formel I angegebene Bedeutung hat,

i) den erhaltenen Ketoester der Formel X durch Hydrolyse überführt in eine Verbindung der Formel I, worin $R^2$ die zur Formel I genannte Bedeutung hat und

$R^1$ Wasserstoff und

$X^1$ und $Y^1$ sowie $X^2$ und $Y^2$ jeweils zusammen Sauerstoff darstellen,

j) gegebenenfalls eine Verbindung der Formel I, worin $X^1$, $X^2$, $Y^1$, $Y^2$ sowie $R^2$ die zur Formel I in Anspruch 1 angegebene Bedeutung haben und $R^1$ Wasserstoff bedeutet, zu einer Verbindung der Formel I, worin $X^1$, $X^2$, $Y^1$, $Y^2$ sowie $R^2$ die zur Formel I in Anspruch 1 angegebenen Bedeutungen haben, wobei $R^1$ jedoch nicht Wasserstoff bedeutet, verestert,

k) und gegebenenfalls eine Verbindung der Formel I, worin $X^1$, $X^2$, $Y^1$, $Y^2$ sowie $R^2$ die zur Formel I in Anspruch 1 angegebenen Bedeutungen haben und $R^1$ Wasserstoff bedeutet, in ein physiologisch verträgliches Metall oder Aminsalz überführt.

3. Verfahren zur Herstellung von Arzneimitteln, dadurch gekennzeichnet, dass man eine Verbindung der in Anspruch 1 genannten Formel I, gegebenenfalls mit üblichen pharmazeutischen Trägern und/oder Stabilisatoren, in eine therapeutisch geeignete Darreichungsform bringt.

0008076

Europäisches Patentamt

# EUROPÄISCHER RECHERCHENBERICHT

Nummer der Anmeldung
EP 79102759.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P | DE - A1 - 2 828 075 (UPJOHN)<br>+ Ansprüche 1, 31 und 32; Seiten 80 - 85 +<br>-- | 1,4,7, 10, 11 |
| | FR - A - 2 376 134 (UPJOHN, 28 - 07 - 78)<br>+ Anspruch 1 +<br>-- | 1,4,7, 10,11 |
| A | DE - A1 - 2 632 097 (HOECHST)<br>+ Ansprüche 1 - 3 +<br>-- | 1,4,7, 9,10,11 |
| A | DE - A1 - 2 702 370 (HOECHST)<br>+ Seiten 1 - 11 +<br>-- | 1,4,7, 9,10,11 |
| A | DE - A1 - 2 617 338 (MAY & BAKER)<br>+ Anspruch 1, Seiten 13 -15 +<br>-- | 1,4,7, 9,10,11 |
| A | DE - A - 2 365 035 (ONO)<br>+ Anspruch 1 +<br>-- | 1,4,7, 9,10,11 |
| A | US - A - 4 052 512 (HAYASHI)<br>+ Spalten 3 - 5 +<br>-- | 1,4,7, 9,10,11 |
| A | DE - A1 - 2 460 285 (ONO)<br>+ Anspruch 1 +<br>-- | 1,4,7, 10,11 |
| A | DE - A1 - 2 700 021 (ONO)<br>+ Ansprüche 1 und 8 +<br>-- | 1,4,7, 9,10,11 |
| A | DE - A1 - 2 736 446 (ONO)<br>+ Anspruch 1 +<br>-- | 1,4,7, 10,11 |

KLASSIFIKATION DER ANMELDUNG (Int.Cl. 3)

C 07 C 177/00
C 07 C 47/267
C 07 D 319/06
A 61 K 31/557

RECHERCHIERTE SACHGEBIETE (Int. Cl. 3)

C 07 C 177/00
C 07 C 47/267
C 07 D 319/06
A 61 K 31/557

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 12-10-1979 | PETROUSEK |

Europäisches Patentamt

**EUROPÄISCHER RECHERCHENBERICHT**

Nummer der Anmeldung
0008076
EP 79102759.2

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | DE - A1 - 2 631 894 (ONO)<br>+ Anspruch 1 +<br>-- | 1,4,7,<br>10,11 | |
| P,A | DE - A1 - 2 825 440 (ONO)<br>+ Anspruch 1 +<br>-- | 1,4,7,<br>10,11 | |
| D,A | DE - A1 - 2 546 313 (HOECHST)<br>+ Ansprüche 1 - 3 +<br>---- | 1,4,7,<br>9,10,<br>11 | |
| | | | RECHERCHIERTE SACHGEBIETE (Int. Cl.3) |

EPA Form 1503.2 06.78